(19) **Europäisches Patentamt**

**European Patent Office**

**Office européen des brevets**

(11) **EP 1 650 563 A1**

(12) **EUROPEAN PATENT APPLICATION**
published in accordance with Art. 158(3) EPC

(43) Date of publication:
26.04.2006 Bulletin 2006/17

(51) Int Cl.:
*G01N 33/50* (1980.01)

(21) Application number: 04771494.4

(86) International application number:
PCT/JP2004/011509

(22) Date of filing: 04.08.2004

(87) International publication number:
WO 2005/012903 (10.02.2005 Gazette 2005/06)

(84) Designated Contracting States:
DE FR GB SE

(30) Priority: 04.08.2003 JP 2003285957

(71) Applicant: KIKKOMAN CORPORATION
Noda-shi,
Chiba 278-8601 (JP)

(72) Inventors:
• SUZUKI, Shigeya,
Kikkoman Corporation
Noda-shi, Chiba 278-8601 (JP)

• HATTORI, Noriaki,
Kikkoman Corporation
Noda-shi, Chiba 278-8601 (JP)
• KURATSUNE, Hirohiko
Toyonaka-shi, Osaka 560-0044 (JP)

(74) Representative: Kramer - Barske - Schmidtchen
Radeckestrasse 43
81245 München (DE)

(54) **METHOD OF JUDGING FATIGUE**

(57) A method of judging fatigue, which comprises judging fatigue by using, as an index, a value obtained by quantitatively analyzing an adenine nucleotide in a sample to be measured.

## FIG. 2

EP 1 650 563 A1

**Description**

Technical Field

The present invention relates to a novel method of judging fatigue. More specifically, the present invention relates to a method of judging fatigue using, as an index, a value obtained by quantitatively analyzing an adenine nucleotide in a sample to be measured.

Background of the Invention

Although the definition and general idea of fatigue vary, it is considered in general that fatigue is a state of showing temporally qualitative or quantitative reduction of physical and mental working abilities, which can be seen when a physical or mental load is continuously applied. In addition, it is considered in general that chronic fatigue syndrome, muscular fatigue, mental fatigue, immunological fatigue, fatigue accompanied by endocrinological abnormality, thermal fatigue and the like are included in the fatigue.

Among the above-described fatigues, the chronic fatigue syndrome (hereinafter referred to as "CFS") is a general idea of a disease in which healthy social life becomes unable to spend due to chronic fatigued feeling of unknown cause as one syndrome for the purpose of revealing its cause of disease and morbid state. There are many cases in which the disease is suddenly developed one day triggered by a case in which a person who has been spending healthy life caught cold or the like, wherein symptoms such as slight fever, headache, lymph node enlargement, muscle pain, feeling of exhaustion, disturbance of ability to think and power of concentration, depressive symptom and sleeplessness are observed for a prolonged period of time together with severe systemic fatigued feeling of unknown cause (e.g., H. Kuratsune, *Igaku no Ayumi* (*Progress of Medical Science*), February 1, 2003, Vol. 204, No. 5, pp. 381-386).

In 1999, a fatigue examination research group of the Ministry of Health and Welfare, Japan (presently, the Ministry of Health, Welfare and Labor) has carried out epidemiological examination of fatigue on 4,000 male and female subjects of 15 to 65 years old and revealed that 35.8% of the subjects admitted chronic fatigue which continued for six months or more, and reduction of working abilities due to fatigue was found in half the number or more of the persons thereof in comparison with the previous years. In addition, it was also proved that about 0.3% of Japanese people satisfy the diagnostic standard of CFS which causes serious difficulties in the daily life and social life (e.g., Masumi Minowa, "Actual state and Risk Factors of Fatigue in Local Society", welfare science research funds subsidy, health science overall research project "Research on the actual condition of fatigue, and studies on fatigue relieving means for health promotion", the year of Heisei 11, Research achievement report, 2000, pp. 19-38).

Accordingly, chronic fatigue is a serious social problem also from the viewpoint of economic loss in the present society, and elucidation of its cause and establishment of appropriate diagnostic method thereof and therapeutic method are in demand. Regarding the cause of the disease, examinations have been made from various points of view including viral infection as well as various immune abnormality, energy metabolism abnormality, mental and psychological causes and the like, but it is not revealed yet (e.g., Teruo Kitani, "CFS no Gainen (Concept of CFS)", *Rinsho Kagaku* (*Clinical Chemistry*), 1993, Vol. 29, No. 6, pp. 663-668).

In addition, in recent years, medicines, quasi drugs, health foods and the like have been developed and are on sale declaring their effect to relieve fatigue. However, since established judging methods of fatigue are not present, it is the present situation that actual proof the effect is obscure. Also, from these viewpoints, concern has been directed toward the development of a judging method which can sort out kinds of fatigue and quantify respective fatigue.

Conventionally, a judging method which uses an inquiry table or the like based on subjectivity of the person was the main stream of the method of judging fatigue. However, since it is greatly influenced by the subjectivity of the person, there frequently was a case in which fatigue cannot be judged correctly. An objective fatigue judging method which is not affected by the subjectivity of the person is in demand.

As an objective fatigue judging method, a method in which a monotonous work is repeatedly carried out, and reduction of working ability thereon is examined or the like has been developed (e.g., Osami Kajimoto, *Igaku No Ayumi* (*Progress of Medical Science*), February 1, 2003, Vol. 204, No. 5, pp. 377-380). However, it is difficult to synthetically judge fatigue by this fatigue judging method alone, for example, because it is influenced by factors other than the working ability and individual difference. It is considered that it is necessary to evaluate fatigue objectively also from the physiological or biochemical point of view.

As the change of physiological function caused by fatigue, recovery delay of heart rate and respiration, increase of stimulation threshold of tendon reflex, reduction of blood pressure controlling function at the time of postural change, expansion of two-point distance of the skin, reduction of flicker distinguishing ability and the like have been exemplified. In addition, regarding blood properties, increase of lactic acid and pyruvic acid and reduction of pH have been shown, and reduction of pH has been reported in the case of saliva, and increase of excretion acceleration of protein and Donaggio reaction-positive substance in the case of urine. There is a method in which lactic acid concentration in blood is measured or the like as a fatigue judging method which measures these changes (e.g., JP-A-05-003798), but this is limited to muscle fatigue so that it is difficult to judge fatigue at individual level synthetically by this method alone.

Also, conventionally, in order to evaluate physiological activities at the cell or tissue level, quantitative analysis of them using an adenine nucleotide as the index has been carried out. Although there are reports on the relationship between these indexes and fatigue, partial muscle fatigue is judged by quantitatively analyzing adenine nucleotides in muscle tissues in these reports, and the relationship is not used as an index for judging fatigue at the individual level (e.g., K. Sahlin, "Plasma hypoxanthine and ammonia in humans during prolonged exercise", *Eur. J. Appl. Physiol.*, 1999, Vol. 80, pp. 417-422).

Also, there is a case in which a load was added to muscles of patients of chronic fatigue syndrome and healthy persons, and a difference was found between partial muscle tissues in terms of a change at the ATP level (e.g., Roger Wong, "Skeletal muscle metabolism in the chronic fatigue syndrome. *In vivo* assessment by $^{31}$P nuclear magnetic resonance spectroscopy.", *Chest*, December, 1992, Vol. 102, No. 6, pp. 1716-1722), but it is dangerous to judge a fatigued state at the individual level based on a partial loading. Although it may be useful as an index of the chronic fatigue syndrome, it is difficult to judge fatigue at the individual level synthetically.

Also, as a method of measuring ATP, ADP and AMP in muscles, there is a method which uses a collected muscle tissue as the measuring subject, but it is difficult to put this into practical use as a general clinical test. In addition, a method of measuring radiation-labeled phosphorus ($^{31}$P) by NMR and the like are also known, but since a radiation is used, it is considered that its realization is difficult, and the muscle tissue is not suited as a measuring sample. Accordingly, in order to judge fatigue at the individual level synthetically, a method which uses a clinically applicable measuring sample and uses an objective index is in demand.

Disclosure of the Invention

An object of the present invention is to provide a novel method of judging fatigue.

The present inventors have conducted intensive studies on the above-described problems and, as a result, found that fatigue can be judged by using, as an index, a value obtained by quantitatively analyzing an adenine nucleotide in a sample to be measured, thereby accomplishing the present invention.

That is, the present invention relates to the following (1) to (7).

(1) A method of judging fatigue, which comprises judging fatigue by using, as an index, a value obtained by quantitatively analyzing an adenine nucleotide in a sample to be measured.

(2) The method according to (1), wherein the value obtained by quantitatively analyzing an adenine nucleotide is associated with fatigue.

(3) A method of analyzing a sample to be measured obtained from a subject for judging fatigue, which comprises quantitatively analyzing an adenine nucleotide in the sample to be measured, and associating the value obtained by the quantitative analysis with fatigue.

(4) The method according to any one of (1) to (3), wherein the fatigue is judged by comparing a value obtained by quantitatively analyzing an adenine nucleotide in a sample to be measured obtained from a subject, with a value obtained by quantitatively analyzing an adenine nucleotide in a sample to be measured obtained from a healthy person.

(5) The method according to any one of (1) to (4), wherein the adenine nucleotide is at least one selected from the group consisting of AMP, ADP and ATP.

(6) The method according to any one of (1) to (5), wherein the value obtained by quantitatively analyzing an adenine nucleotide is at least one selected from the group consisting of concentration, construction ratio, concentration ratio and adenylate energy charge.

(7) The method according to any one of (1) to (6), wherein the sample to be measured is blood.

(8) The method according to any one of (1) to (7), wherein the fatigue is chronic fatigue syndrome.

The present invention is described below in detail.

The sample to be measured in the present invention includes various body fluids such as saliva, urine, blood (total blood, serum, plasma, erythrocyte, monocyte, leukocyte, lymphocyte, etc.), sweat and tears, various tissues such as organ tissues, and the like of the subjects, although not particularly limited thereto. Also, the sample of the present invention to be measured may be used by diluting with water or an aqueous solution such as a buffer or after treatment such as centrifugation, so long as it does not exert influence upon the quantitative analysis of adenine nucleotides. In addition, additives such as a surfactant, an antiseptic, a stabilizer and a reaction accelerator may be added thereto, if necessary.

The method for quantitatively analyzing adenine nucleotides in the present invention includes methods which use an enzyme such as a method which uses firefly luciferase and pyruvate orthophosphate kinase and a method which uses firefly luciferase, adenylate kinase and pyruvate kinase; a method which uses liquid chromatography; a method which uses an electrochemical technique; and the like, although not particularly limited thereto.

The adenine nucleotide to be quantitatively analyzed in the present invention includes AMP, ADP, ATP, cyclic AMP and the like, although not particularly limited thereto. In this connection, although it is possible to carry out quantitative analysis of nucleic acid-related substances other than adenine nucleotides, including various nucleotides such as inosinic acid, inosine and hypoxanthine, nucleosides, nucleic acid bases and the like, it is preferable to quantitatively analyze adenine nucleotides in the present invention.

The value obtained by quantitatively analyzing adenine nucleotides includes concentration of respective adenine nucleotides, concentration of total adenine nucleotides, concentration ratio of respective adenine nucleotides, construction ratio of respective adenine nucleotides, adenylate energy charge (hereinafter referred to as "AEC") and the like, although not particularly limited thereto.

The concentration of total adenine nucleotides includes, for example, a concentration of ATP + ADP + AMP, but concentration of other adenine nucleotide derivatives such as cyclic AMP may be included therein.

The concentration ratio of respective adenine nucleotides includes, for example, a concentration ratio of respective adenine nucleotide such as ATP/AMP or ADP/AMP.

The construction ratio of respective adenine nucleotides includes, for example, a concentration ratio of respective adenine nucleotides (ATP, ADP, AMP) based on the total adenine nucleotide concentration (concentration of ATP + ADP + AMP).

The AEC is a value obtained by a calculation formula shown in the following formula (1). This is a value which shows the ratio of actually attached phosphoric acid(s) among two phosphoric acids which can be attached to AMP, and is a value that shows the energy charge. When all of AMP and ADP become ATP, the AEC becomes 1.

$$AEC = (ATP + 0.5 \times ADP)/(ATP + ADP + AMP) \qquad (1)$$

According to the present invention, fatigue can be judged by using, as an index, a value obtained by quantitatively analyzing an adenine nucleotide in a sample to be measured. For example, fatigue can be judged by associating the value obtained by quantitatively analyzing adenine nucleotide with fatigue.

Specifically, fatigue can be judged by comparing a value obtained by quantitatively analyzing an adenine nucleotide in a sample to be measured obtained from a subject, with a value obtained by quantitatively analyzing an adenine nucleotide in a sample to be measured obtained from a healthy person. In this comparison with the value obtained by quantitatively analyzing an adenine nucleotide of a healthy person, the comparison may be carried out by directly collecting a sample to be measured from a healthy person, or may be carried out with a standard value data calculated from the values obtained in advance by quantitatively analyzing an adenine nucleotide of a healthy person or with a value obtained from a standard sample prepared based on the data. The data may be data obtained from two or more healthy persons, or data of the subject himself obtained in advance when the subject himself was in a healthy state.

More specifically, since the abundance ratio and abundance of respective adenine nucleotides show abnormal values under a fatigued state in comparison with a healthy state, the fatigued state and degree of fatigue of the subject can be judged by associating them as indexes with fatigue. Specifically, when at least one value selected from concentration of respective adenine nucleotides, concentration of total adenine nucleotides, concentration ratio of respective adenine nucleotides, construction ratio of respective adenine nucleotides and AEC in a sample to be measured of a subject is compared with that of a healthy person, and when there is a significant difference between the data of both cases, it can be judged that the subject is under a fatigued state. In this case, the significance level P is preferably $P < 0.05$ (5%), more preferably $P < 0.01$ (1%). Since there is a case in which an abnormal value of the data under a fatigued state is caused by reduction of ATP as the energy source in the living body, or on the contrary, there is a case in which it is caused by accumulation and increase of ATP which cannot be used as the energy source in the living body, this abnormal value can be used in the judgment of the present invention in both cases in which the data of the subject are significantly higher and significantly lower than the data of the healthy person.

Although there is a case in which difference in the degree of fatigue becomes unclear by influence of a body fluid and the like when concentration of respective adenine nucleotides alone is calculated, the difference in the degree of fatigue can be shown more clearly by calculating concentration of respective adenine nucleotides, concentration of total adenine nucleotides, concentration ratio of respective adenine nucleotides, construction ratio of respective adenine nucleotides and AEC, and using them in combination as the indexes, so that it becomes possible to increase accuracy of the judgment of fatigue.

In addition, fatigue of a subject can also be judged by using a kit which comprises a reagent and/or instrument necessary for the quantitative analysis of an adenine nucleotide, and a standard value data calculated from the values obtained in advance by quantitatively analyzing an adenine nucleotide of a healthy person and/or a standard sample prepared based on the data. These reagent and instrument and the standard value data and standard sample may be used as a combination of respectively independent kits, or used by including in the same kit.

As the kinds of fatigue to be judged by the present invention, chronic fatigue syndrome, mental fatigue, immunological fatigue, fatigue accompanied by endocrinological abnormality, thermal fatigue and the like are exemplified, although not particularly limited thereto. In addition, according to the present invention, it is also possible to judge the degree of these fatigues by judging from the degree of difference between a subject and a healthy person in terms of the concentration of respective adenine nucleotide, concentration of total adenine nucleotides, concentration ratio of respective adenine nucleotide, construction ratio of respective adenine nucleotide and AEC.

As the subject in the present invention, it may be a patient possibly having a disease based on the above-described fatigue, or a subject who does not have such a disease but requires confirmation of own heath conditions. Accordingly, according to the judging method of the present invention, it is possible to screen patients of a disease based on fatigue and to judge degree of the disease, if necessary, or to screen subjects who have no diseases based on fatigue but are fatigued and to judge degree of the fatigue, if necessary. Also, when a subject is judged not to be a fatigued state according to the judging method of the present invention, it can be considered that the subject is healthy. In addition, when a subject felt fatigue but was judged not to be a fatigued state according to the judging method of the present invention, it can be considered that the subject is suffering from a disease other than fatigue.

As the time for judging fatigue according to the present invention, a process of accumulating fatigue, the time when fatigue is accumulated, a process of recovering from fatigue and the like can be exemplified, although not particularly limited thereto.

The subject whose fatigue is judged according to the present invention has been described based on human, but it may be an animal other than human, so long as the judging method of the present invention can be applied.

The characteristics of the present invention are described in detail based on Examples, but the technical scope of the present invention is not limited thereto.

Brief Description of the Drawings

Fig. 1 shows calibration curves by respective reagents for adenine nucleotide fractional determination.
Fig. 2 shows comparison of respective adenine nucleotide concentrations in total blood.
Fig. 3 shows comparison of respective adenine nucleotide concentrations in plasma.
Fig. 4 shows comparison of respective adenine nucleotide construction ratios in plasma.
Fig. 5 shows comparison of respective adenine nucleotide concentrations in erythrocyte.

Best Mode for Carrying Out the Invention

Example 1

Quantitative analysis of adenine nucleotides in body fluid or blood of healthy parson and CFS patient:

Body fluid or blood was collected from healthy parsons and CFS patients, quantitative analysis of adenine nucleotides was carried out and the results were compared.

As the reagents for adenine nucleotide fractional determination, the following 3 kinds (a. for ATP measurement, b. for ATP + AMP measurement, c. for ATP + ADP + AMP measurement) were prepared and used.

Compositions of respective luminescent reagents are as follows.

a. For ATP measurement

2 mM MES, 40 mM BES, 5% sucrose, 1 mM EDTA-2Na, 0.2 U/ml adenosine phosphate deaminase, 10.1 mM magnesium acetate, 0.1 mM dithiothreitol, 0.36 GLU/ml luciferase, 0.4% bovine serum albumin, 4.2 mM potassium phosphoenolpyruvate, 0.6 mM potassium pyrophosphate, 1.6 mM luciferin (pH 8.0).

b. For ATP + AMP measurement

2 mM MES, 40 mM BES, 5% sucrose, 1 mM EDTA·2Na, 0.2 U/ml adenosine phosphate deaminase, 10.1 mM magnesium acetate, 0.1 mM dithiothreitol, 0.36 GLU/ml luciferase, 0.4% bovine serum albumin, 4.2 mM potassium phosphoenolpyruvate, 0.6 mM potassium pyrophosphate, 1.6 mM luciferin, 2.68 U/ml pyruvate orthophosphate dikinase (pH 8.0).

c. For ATP + ADP + AMP measurement

2 mM MES, 40 mM BES, 5% sucrose, 1 mM EDTA-2Na, 0.2 U/ml adenosine phosphate deaminase, 10.1 mM magnesium acetate, 0.1 mM dithiothreitol, 0.36 GLU/ml luciferase, 0.4% bovine serum albumin, 4.2 mM potassium phosphoenolpyruvate, 0.6 mM potassium pyrophosphate, 1.6 mM luciferin, 2.68 U/ml pyruvate orthophosphate dikinase, 200 U/ml pyruvate kinase (pH 8.0).

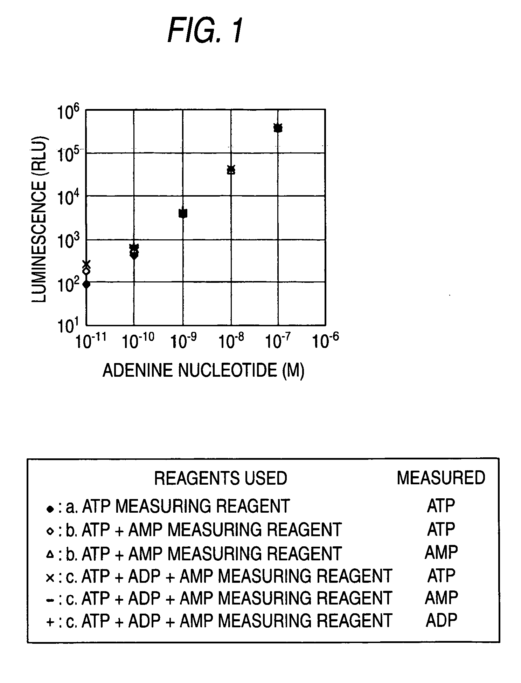

Calibration curves of respective measuring reagents were prepared in the following manner. After 0.1 ml of each concentration of respective adenine nucleotides was added to 0.1 ml of each of 3 kinds (a. for ATP measurement, b. for ATP + AMP measurement, c. for ATP + ADP + AMP measurement) of adenine nucleotide fractional determination reagents, the luminescence at that time was measured using Lumitester K-210 (manufactured by Kikkoman Corporation). The results are shown in Fig. 1.

Concentration of AMP in each sample was calculated by the following formula (2):

$$AMP = (ATP + AMP) - ATP \qquad (2)$$

Concentration of ADP was calculated by the following formula (3):

$$ADP = (ATP + ADP + AMP) - (ATP + AMP) \qquad (3)$$

Preparation of measuring samples and measuring methods are as follows.

1. Total blood

Approximately 2 ml of peripheral blood was collected using a blood collection tube containing EDTA, and 0.3 ml thereof was collected. The same volume of 10% TCA was added thereto and thoroughly stirred using a Vortex mixer, followed by centrifugation at 15,000 rpm for 5 minutes, and then the supernatant was diluted 10,000-fold with 50 mM Tris/HCl buffer (pH 7.5) (total blood dilution). As the measurement of adenine nucleotide quantity, 0.1 ml of the measuring sample (total blood dilution) was added to 0.1 ml of each of 3 kinds (a. for ATP measurement, b. for ATP + AMP measurement, c. for ATP + ADP + AMP measurement) of adenine nucleotide fractional determination reagents, and the luminescence at that time was measured using Lumitester K-210. As the blank, 50 mM Tris/HCl buffer (pH 7.5) was used, and as the standard, $1 \times 10^{-7}$ M ATP diluted with 50 mM Tris/HCl buffer (pH 7.5) was used.

2. Plasma

Approximately 2 ml of peripheral blood was collected using a blood collection tube containing EDTA and slowly mixed, followed by centrifugation at 3,000 rpm for 10 minutes. After 0.5 ml of the upper layer (plasma) was collected, 0.5 ml of 10% TCA was added thereto and thoroughly stirred, followed by centrifugation at 15,000 rpm for 5 minutes. The resulting supernatant diluted 100-fold with 50 mM Tris/HCl buffer (pH 7.5) (plasma dilution). The adenine nucleotide quantity was measured in the same manner as the case of total blood.

3. Erythrocyte

Approximately 2 ml of peripheral blood was collected using a blood collection tube containing EDTA and mixed, followed by centrifugation at 3,000 rpm for 10 minutes. Then, 0.1 ml was collected from the lower layer (erythrocyte) and diluted 10-fold (erythrocyte 10-fold dilution) by adding to 0.9 ml of 1% FBS-containing PBS buffer which had been dispensed in advance. Then, 0.5 ml was collected therefrom, and 0.5 ml of 10% TCA was added thereto, followed by thoroughly stirring and centrifugation at 15,000 rpm for 5 minutes. Then, the mixture was diluted 1,000-fold with 50 mM Tris/HCl buffer (pH 7.5) (erythrocyte dilution). The adenine nucleotide quantity was measured in the same manner as the case of total blood. In addition, the erythrocyte dilution was diluted 900-fold with 1% FBS-containing PBS, and the number of hemocytes was measured using a hemocytometer.

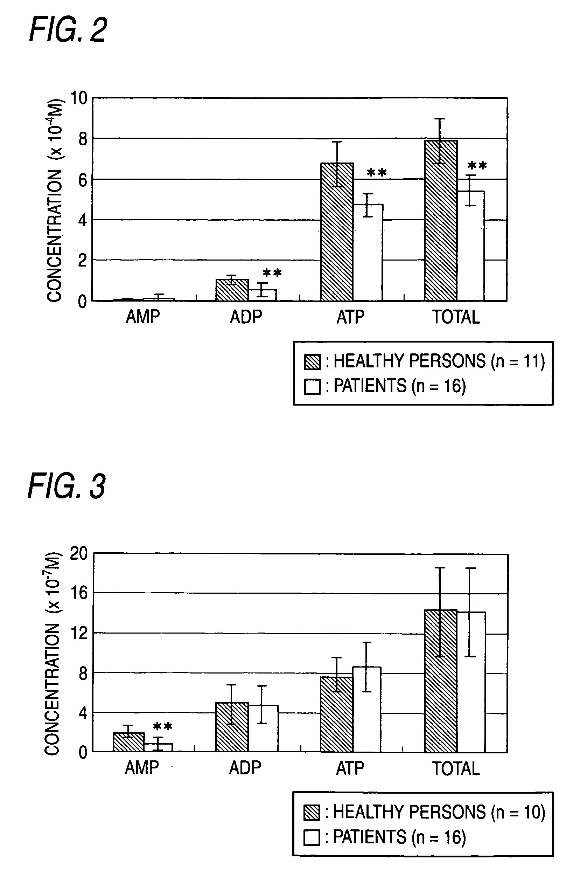

The results and discussion are shown below. Also, the symbol ** in Figs. 2 to 5 represents that there is a significant difference with a significance level of $P < 0.01$. Also, the symbol * in Figs. 2 to 5 represents that there is a significant difference with a significance level of $P < 0.05$. Also, the term "Total" in Fig. 2 to Fig. 5 represents a total adenine nucleotide concentration. The total adenine nucleotide concentration represents a concentration of ATP + ADP + AMP.

1. Total blood

Results of respective adenine nucleotide concentrations are shown in Fig. 2. The ADP, ATP and total adenine nucleotide concentrations in the CFS patients were significantly lower than those in the healthy persons at a reliability of 99%.

2. Plasma

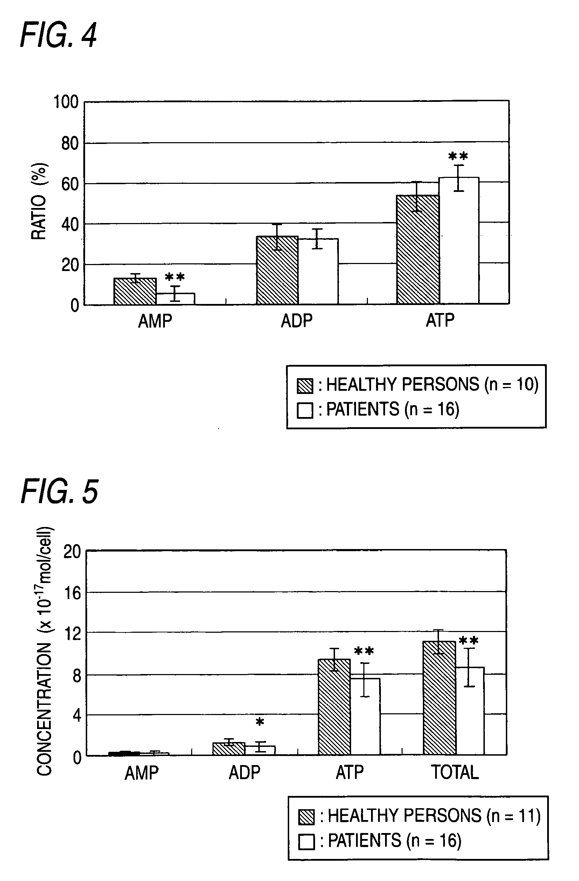

Results of respective adenine nucleotide concentrations are shown in Fig. 3. The concentration of AMP in the CFS patients was significantly lower than that in the healthy persons at a reliability of 99%.
Results of respective adenine nucleotide construction ratios are shown in Fig. 4. The construction ratio of AMP in the CFS patients was significantly lower than that in the healthy persons at a reliability of 99%. Also, the construction ratio of ATP in the CFS patients was significantly higher than that in the healthy persons at a reliability of 99%.

3. Erythrocyte

Results of respective adenine nucleotide concentrations are shown in Fig. 5. The ATP and total adenine nucleotide concentrations in the CFS patients were significantly lower than those in the healthy persons at a reliability of 99%.

4. AEC

When AEC in plasma was calculated, it was $0.78 \pm 0.05$ in the CFS patients, and $0.70 \pm 0.04$ in the healthy persons. When the data were statistically analyzed, AEC in the CFS patients was significantly higher than that in the healthy persons at a reliability of 99%.
Based on these results, it was shown that chronic fatigue can be judged when the values obtained by quantitatively analyzing adenine nucleotides were used as the indexes.
While the invention has been described in detail and with reference to specific embodiments thereof, it will be apparent to one of skill in the art that various changes and modifications can be made therein without departing from the spirit and scope thereof.
This application is based on Japanese application No. 2003-285857 filed on August 4, 2003, the entire contents of which are incorporated hereinto by reference. All references cited herein are incorporated in their entirety.

Industrial Applicability

According to the present invention, fatigue can be judged by using, as an index, a value obtained by quantitatively analyzing an adenine nucleotide in a sample to be measured.

**Claims**

1.   A method of judging fatigue, which comprises judging fatigue by using, as an index, a value obtained by quantitatively analyzing an adenine nucleotide in a sample to be measured.

2.   The method according to claim 1, wherein the value obtained by quantitatively analyzing an adenine nucleotide is associated with fatigue.

3.   A method of analyzing a sample to be measured obtained from a subject for judging fatigue, which comprises quantitatively analyzing an adenine nucleotide in the sample to be measured, and associating the value obtained by the quantitative analysis with fatigue.

4.   The method according to any one of claims 1 to 3, wherein the fatigue is judged by comparing a value obtained by quantitatively analyzing an adenine nucleotide in a sample to be measured obtained from a subject, with a value obtained by quantitatively analyzing an adenine nucleotide in a sample to be measured obtained from a healthy person.

5. The method according to any one of claims 1 to 4, wherein the adenine nucleotide is at least one selected from the group consisting of AMP, ADP and ATP.

6. The method according to any one of claims 1 to 5, wherein the value obtained by quantitatively analyzing an adenine nucleotide is at least one selected from the group consisting of concentration, construction ratio, concentration ratio and adenylate energy charge.

7. The method according to any one of claims 1 to 6, wherein the sample to be measured is blood.

8. The method according to any one of claims 1 to 7, wherein the fatigue is chronic fatigue syndrome.

# FIG. 1

| REAGENTS USED | MEASURED |
|---|---|
| ● : a. ATP MEASURING REAGENT | ATP |
| ◇ : b. ATP + AMP MEASURING REAGENT | ATP |
| △ : b. ATP + AMP MEASURING REAGENT | AMP |
| × : c. ATP + ADP + AMP MEASURING REAGENT | ATP |
| – : c. ATP + ADP + AMP MEASURING REAGENT | AMP |
| + : c. ATP + ADP + AMP MEASURING REAGENT | ADP |

## FIG. 2

## FIG. 3

## FIG. 4

## FIG. 5

## INTERNATIONAL SEARCH REPORT

| International application No. |
| --- |
| PCT/JP2004/011509 |

**A. CLASSIFICATION OF SUBJECT MATTER**
Int.Cl⁷ G01N33/50

According to International Patent Classification (IPC) or to both national classification and IPC

**B. FIELDS SEARCHED**

Minimum documentation searched (classification system followed by classification symbols)
Int.Cl⁷ G01N33/48-33/98

Documentation searched other than minimum documentation to the extent that such documents are included in the fields searched

| | | | |
| --- | --- | --- | --- |
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)
CA

**C. DOCUMENTS CONSIDERED TO BE RELEVANT**

| Category* | Citation of document, with indication, where appropriate, of the relevant passages | Relevant to claim No. |
| --- | --- | --- |
| X/A | Roger WONG et al., "Skeltalo Muscle Metabolism in the Chronic Fatigue Syndrome", CHEST, Vol. 102, No.6, (1992), pages 1716 to 1722 | 1-6,8/7 |
| P,A | McCULLY et al., "Blood Flow and muscle metabolism in chronic fatigue syndrome", Clinical Science, Vol.104, (June 2003), pages 641 to 647 | 1-8 |
| A | FORSYTH et al., "Therapeutic effects of oral NADH on the symptoms of patients with chronic fatigue syndrome", ANNALS OF ALLERGY, ASTHMA, &IMMUNOLOGY, Vol.82, No.2, (1999), pages 185 to 191 | 1-8 |

☐ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
| --- | --- |
| Date of the actual completion of the international search<br>13 September, 2004 (13.09.04) | Date of mailing of the international search report<br>28 September, 2004 (28.09.04) |
| Name and mailing address of the ISA/<br>Japanese Patent Office | Authorized officer |
| Facsimile No. | Telephone No. |

Form PCT/ISA/210 (second sheet) (January 2004)